## Europäisches Patentamt

## European Patent Office

## Office européen des brevets

(11) Veröffentlichungsnummer: **0 023 325**
**B1**

(12) # EUROPÄISCHE PATENTSCHRIFT

(45) Veröffentlichungstag der Patentschrift:
06.04.83

(51) Int. Cl.³: **C 07 C 37/20**, C 07 C 39/16,
B 01 J 31/10

(21) Anmeldenummer: 80104231.8

(22) Anmeldetag: 18.07.80

(54) Katalysator für die Herstellung von Bisphenolen.

(30) Priorität: 31.07.79 DE 2931036

(43) Veröffentlichungstag der Anmeldung:
04.02.81 Patentblatt 81/5

(45) Bekanntmachung des Hinweises auf die Patenterteilung:
06.04.83 Patentblatt 83/14

(84) Benannte Vertragsstaaten:
BE DE FR GB IT NL

(56) Entgegenhaltungen:
DE-A-2 164 339
FR-A-1 575 847
GB-A-842 209
US-A-3 172 916
US-A-3 326 866

Die Akte enthält technische Angaben, die nach dem Eingang der Anmeldung eingereicht wurden und die nicht in dieser Patentschrift enthalten sind.

(73) Patentinhaber: BAYER AG, Zentralbereich Patente, Marken und Lizenzen, D-5090 Leverkusen 1, Bayerwerk (DE)

(72) Erfinder: Heydenreich, Frieder, Dr., Kohlstrasse 34, D-4030 Ratingen 6 (DE)
Erfinder: Wulff, Claus, Dr., Kaiserstrasse 248, D-4150 Krefeld (DE)
Erfinder: Klein, Lothar, Dr., Max-Beckmann-Strasse 59, D-5090 Leverkusen 1 (DE)
Erfinder: Meissner, Hans-Jürgen, Dr., Scheibler Strasse 101, D-4150 Krefeld (DE)
Erfinder: Bachem, Norbert, Dr., Jentgesallee 43, D-4150 Krefeld (DE)

Katalysator für die Herstellung von Bisphenolen

Bei der technischen Herstellung von Bisphenolen aus Phenolen und Carbonylgruppen-haltigen Verbindungen verwendet man saure Ionenaustauscher als Katalysatoren. Ihre Aktivität kann durch Cokatalysatoren gesteigert werden. Diese Cokatalysatoren sind im allgemeinen Mercaptoverbindungen, die teilweise ionogen an den Ionenaustauscher gebunden sind (vergl. FR-PS 1 575 847). Beispielsweise hat man verwendet: Mercaptopropionsäure (US-PS 2 468 982), phenollösliche Mercaptane (GB-PS 1 185 102) und Thiazolidin (DE-A 2 164 339).

Diese Cokatalysatoren sind in der Regel nicht oder nur ionogen an den Ionenaustauscher gebunden und daher im Reaktionsmedium löslich. Sie gelangen bei der Aufarbeitung zumindest teilweise in das Reaktionsprodukt und verschlechtern dessen Qualität.

Das US-Patent 3 172 916 beschreibt einen Katalysator für die Herstellung von Bisphenolen bestehend aus einem Styrol/Divinylbenzol-Copolymerisat, in das Sulfochloridgruppen eingeführt sind, die ihrerseits teilweise mit Zinn- und Salzsäure zu Mercaptogruppen reduziert und teilweise zu Sulfonsäuregruppen verseift worden sind. Diese Produkte sind technisch nicht reproduzierbar herzustellen.

Der Erfindung liegt die Erkenntnis zugrunde, dass Cokatalysatoren ohne Verlust von Aktivität durch covalente Bindungen an das Ionenaustauschergerüst gebunden werden können. Die Cokatalysatoren sind dann unlöslich und gelangen nicht mehr ins Reaktionsprodukt.

Gegenstand der Erfindung ist somit ein Katalysator für die Herstellung von Bisphenolen aus Phenolen und Carbonylgruppen enthaltenden Verbindungen; bestehend aus einem Styrol/Divinylbenzol-Copolymerisat, in das Sulfochloridgruppen eingeführt sind, die ihrerseits teilweise mit einem Mercaptoalkylamin umgesetzt und teilweise zu Sulfonsäuregruppen verseift worden sind.

Der neue Katalysator kann wie folgt hergestellt werden:

Ein vorzugsweise gequollenes Styrol/Divinylbenzol-Copolymerisat wird nach den üblichen Methoden zur Herstellung von aromatischen Sulfochloriden zu einem Sulfochlorid umgesetzt. Als Quellmittel können die bekannten Quellmittel verwendet werden, beispielsweise 1,2-Dichlorethan. Der Substitutionsgrad soll möglichst ≥ 0,9 sein. Vorzugsweise wird für die Herstellung des Sulfochlorids Chlorsulfonsäure verwendet.

Nach erfolgter Chlorsulfonierung wird die überschüssige Chlorsulfonsäure abgetrennt und etwa 15 mol-% der Sulfochloridgruppen mit dem Mercaptoamin zu dem Sulfonamid umgesetzt. Anschliessend werden vorzugsweise mit einer Wasserdampfdestillation die verbliebenen Sulfochloridgruppen zu Sulfonsäuregruppen hydrolysiert, sowie noch vorhandene Reste von Quell- oder Lösungsmitteln ausgetrieben. Das Trocknen des wasserfeuchten Katalysators kann thermisch oder vorzugsweise durch Entwässern mit einem Phenol erfolgen.

Zur Herstellung des Katalysators geeignete Styrol/Divinylbenzol-Copolymerisate sind an sich bekannt, bevorzugt sind Produkte mit einem Divinylbenzolgehalt von 0,2 bis 25%.

Mercaptoalkylamine im Sinne der Erfindung sind Verbindungen, die zugleich eine oder mehrere primäre oder sekundäre Aminogruppen und mindestens eine Mercaptogruppe tragen. Mercaptoarylamine können prinzipiell ebenfalls zur Herstellung der Katalysatoren verwendet werden, allerdings sind Mercaptoalkylamine bevorzugt. Besonders geeignete Mercaptoalkylamine sind beispielsweise 2-Mercaptoethylamin, 2-Mercaptoisopropylamin, 3-Mercaptobutylamin und Mercaptopentylamin.

Man kann zur Herstellung des Katalysators auch von sulfonierten Ionenaustauscherharzen ausgehen und wie folgt arbeiten:

Das getrocknete Sulfonsäuregruppen-haltige Ionenaustauscherharz wird in 1,2-Dichlorethan aufgenommen, das als Quell- und Rührmedium dient. Unter Kochen am Rückfluss wird Thionylchlorid zugegeben und die entstandene Salzsäure und das ebenfalls gebildete Schwefeldioxid abgeleitet. Ein befriedigender Umsatz wird erst dann erzielt, wenn katalytische Mengen Pyridin zugegeben werden. Nach Abschluss der Umsetzung mit Thionylchlorid wird das überschüssige Thionylchlorid entfernt und die Umsetzung mit dem Mercaptoamin sowie die abschliessende Hydrolyse angeschlossen.

Nach dem Trocknen, das thermisch oder vorzugsweise durch Entwässern mit Phenol erfolgen kann, erhält man einen Katalysator hoher Aktivität. Auch nach 50 Tagen sinkt die Aktivität noch nicht ab. In den Mutterlaugen kann kein Schwefel nachgewiesen werden, d.h., der Katalysator blutet nicht aus.

Zur Herstellung dieses Katalysators geeignete Ausgangsstufen sind Sulfonsäure-haltige Ionenaustauscherharze mit einer Matrix aus Styrol und Divinylbenzol; der Divinylbenzolgehalt kann dabei von 0,2-25% betragen. Der Sulfonierungsgrad des Harzes ist nicht entscheidend; bevorzugt sind allerdings Harze mit einer möglichst vollständigen Sulfonierung.

Die erfindungsgemässen Katalysatoren können verwendet werden zur Herstellung von Bisphenolen. Als Ausgangsprodukte hierfür geeignete Phenole sind insbesondere Phenol und 2,6-Dimethylphenol und als Ausgangsprodukte geeignete Carbonylgruppen enthaltende Verbindungen sind insbesondere Aceton und Formaldehyd.

Im allgemeinen setzt man 85 bis 99,8 Gew.-Teile des Phenols mit 0,2 bis 15 Gew.-Teilen der Carbonylgruppen enthaltenden Verbindung bei Temperaturen von 45 bis 130° C, gegebenenfalls in Anwesenheit eines Lösungsmittels in einem Katalysatorfest- oder Fliessbett um.

## Beispiel 1

1700 g eines mit 2% Divinylbenzol vernetzten Polystyrols mit einer Restfeuchte (0,1% $H_2O$) werden in 5 l trockenem 1,2-Dichlorethan unter Rühren suspendiert. Bei 0 bis 5°C werden 2 l Chlorsulfonsäure zugegeben, nach beendeter Chlorsulfonierung werden bei Zimmertemperatur unter Rühren 100 g Cysteamin-Hydrochlorid zugegeben. Nachdem die Reaktion beendet ist, wird das Produkt mit Wasserdampf destilliert, wobei die Chlorsulfonsäuregruppen hydrolisiert werden. Das H-Äquivalent des so hergestellten modifizierten Katalysators ist 3,65.

Zum Vergleich wird der Versuch wiederholt, jedoch kein Cysteamin-Hydrochlorid zugegeben. Das H-Äquivalent des so erhaltenen Produktes ist 4,95.

Der erfindungsgemässe modifizierte Katalysator (A) und das mit β-Mercaptopropionsäure als Cokatalysator umgesetzte Vergleichsprodukt (B) werden mit Phenol und Aceton versetzt. Die folgende Tabelle gibt das Ergebnis der beiden Versuche.

| Einsatzmengen | A (in g) | B (in g) |
|---|---|---|
| Phenol | 282,0 | 282,0 |
| Aceton | 14,5 | 14,5 |
| β-Mercaptopropionsäure | — | 0,12 |
| Ionenaustauscherharz | 37,5 | 37,5 |

| Acetonumsatz nach: | A | B |
|---|---|---|
| 1 h | 77,1 | 52,6 |
| 2 h | 87,3 | 66,0 |
| 3 h | 94,5 | 76,3 |
| 4 h | 96,9 | 81,8 |
| 7 h | 98,4 | 91,4 |
| Farbe der Mutterlauge: | hell | dunkel |

## Beispiel 2

14 g eines trockenen Sulfonsäuregruppen-haltigen Harzes aus Styrol und 2% Divinylbenzol (Totalkapazität 4,2 mval/l) werden in 100 ml 1,2-Dichlorethan vorgelegt. Unter Ausschluss von Feuchtigkeit, leichtem Rühren und Kochen am Rückfluss werden langsam 12 g Thionylchlorid zugetropft. Vor Beginn der Thionylchloridzugabe wurden dem Ansatz 0,4 ml Pyridin zugesetzt. Nach beendetem Zutropfen wird noch 2 h nachgerührt.

Der Ansatz wird von dem überschüssigen Thionylchlorid und 1,2-Dichlorethan durch Absaugen über eine Fritte oder Abdestillieren — gegebenenfalls im Vakuum — befreit. Es wird mehrmals mit 1,2-Dichlorethan nachgewaschen, um $SOCl_2$-Reste zu entfernen.

Anschliessend wird wieder in 1,2-Dichlorethan aufgenommen, und unter Rühren bei Raumtemperatur wird 0,8 g Cysteaminhydrochlorid zugegeben. Nach beendeter Reaktion wird der Ansatz einer Wasserdampfdestillation unterworfen, wobei die Sulfochloridgruppen hydrolysiert werden. Das H-Äquivalent des so hergestellten Katalysators beträgt 3,7.

In einem Vergleichsversuch wird der erfindungsgemäss modifizierte Katalysator (A) mit einem nichtmodifizierten Katalysator verglichen, dem β-Mercaptopropionsäure als Cokatalysator zugesetzt wurde.

| Einsatzmengen | A (in g) | B (in g) |
|---|---|---|
| Phenol | 1128 | 1128 |
| Aceton | 58 | 58 |
| β-Mercaptopropionsäure | — | 0,48 |
| Katalysator | 150 | 150 |

| Reaktionstemperatur: 65°C | | |
|---|---|---|
| Acetonumsatz nach 4 h: | 97,5% | 81,0% |
| Farbe der Mutterlauge: | hell | dunkel |

## Patentanspruch

Katalysator für die Herstellung von Bisphenolen aus Phenolen und Carbonylgruppen enthaltenden Verbindungen, bestehend aus einem Styrol/Divinylbenzol-Copolymerisat, in das Sulfochloridgruppen eingeführt sind, die ihrerseits teilweise mit Mercaptoalkylamin umgesetzt und teilweise zu Sulfonsäuregruppen verseift worden sind.

## Claim

A catalyst for the production of bisphenols from phenols and compounds containing carbonyl groups, consisting of a styrene/divinyl benzene copolymer into which are introduced sulfochloride groups which in turn have been partly reacted with mercaptoalkyl amine and partly hydrolysed to form sulfonic acid groups.

## Revendication

Catalyseur pour la préparation de bisphénols à partir de phénols et de composés contenant des groupes carbonyle, consistant en un copolymère styrène/divinylbenzène dans lequel on a introduit des groupes sulfochlorure qu'on a eux-mêmes mis à réagir en partie avec une mercaptoalkylamine et saponifiés en partie en groupes acide sulfonique.